# FASCICULE DE BREVET EUROPEEN

(11) **EP 3 254 538 B1**
(45) Date de publication et mention de la délivrance du brevet: **16.09.2020**
(21) Numéro de dépôt: 16707839.3
(22) Date de dépôt: 27.01.2016
(51) Int. Cl.: H05B 47/155, H05B 47/105, A61M 21/00

(54) **EQUIPEMENT D'ECLAIRAGE A STIMULATION OPTIMALE DES FONCTIONS NON VISUELLES**
BELEUCHTUNGSVORRICHTUNG MIT OPTIMALER STIMULATION VON NICHTVISUELLEN FUNKTIONEN
LIGHTING APPARATUS WITH OPTIMUM STIMULATION OF NON-VISUAL FUNCTIONS

(30) Priorité: 05.02.2015 FR 1550896
(43) Date de publication de la demande: 13.12.2017
(73) Titulaire: Maquet SAS, 45160 Ardon (FR); Inserm, 75013 Paris (FR); ENTPE, 69120 Vaulx en Velin (FR)
(72) Inventeur: DESOUCHES, Jérôme, 45074 Orleans CEDEX 2 (FR); LE BER, David, 45380 Chaingy (FR); GRONFIER, Claude, 69003 Lyon (FR); DUMORTIER, Dominique, 69200 Venissieux (FR); AVOUAC, Pascale, 69100 Villeurbanne (FR); COOPER, Howard, 69660 Collonges au Mont d'Or (FR)
(74) Mandataire: Prugneau, Philippe
(86) Numéro de dépôt international: PCT/FR2016/050164
(87) Numéro de publication internationale: WO 2016/124836

(56) Documents cités:
- EP-A2- 2 051 763
- WO-A1-02/20079
- WO-A2-2008/146220
- US-A1- 2012 095 534

## Description

### Domaine technique

Le domaine de l'invention est celui des équipements d'éclairage notamment utilisés dans les salles d'opération chirurgicales.

L'invention concerne plus particulièrement un équipement d'éclairage notamment pour bloc opératoire comprenant un dispositif d'éclairage avec plusieurs sources de lumière pour fournir une lumière blanche à spectre lumineux accordable et un dispositif de contrôle/commande qui pilote les sources de lumière de façon à moduler le spectre lumineux selon un cycle de modulation répétable sans interruption comprenant une première période de temps où le spectre lumineux est enrichi en rouge, une seconde période de temps où le spectre lumineux est modulé graduellement du rouge vers le bleu, une troisième période de temps où le spectre lumineux est enrichi en bleu, une quatrième période de temps où le spectre lumineux est modulé graduellement du bleu vers le rouge.

### Technique antérieure

La plupart des fabricants de luminaires proposent des luminaires à spectre lumineux accordable. Le changement de spectre se fait en combinant plusieurs sources lumineuses ayant chacune une longueur d'onde propre. Ces sources lumineuses sont généralement des LEDs mais peuvent aussi être des tubes fluorescents. Ces luminaires se composent généralement de trois ou quatre sources lumineuses différentes offrant respectivement les couleurs bleu, vert, rouge et parfois blanc. Ces luminaires permettent généralement la sélection de plusieurs ambiances via un panneau de commande ou un bouton. Certains modes permettent de moduler le spectre au cours du temps, la couleur passant alors du rouge au violet, puis au bleu, puis au vert, etc. La variation de spectre se fait en modulant l'intensité ou la tension aux bornes des sources lumineuses. La modulation de spectre de ces luminaires n'a cependant souvent qu'un but esthétique.

Or, il est connu que les chirurgiens travaillant de nuit dans les hôpitaux soufrent, comme la plupart des travailleurs de nuit, de troubles de la vigilance, de l'humeur, et d'altérations psychomotrices durant leur poste de nuit. Ces déficits sont principalement liés à une mauvaise adaptation (synchronisation) de l'horloge biologique des personnels à une activité nocturne, et sont classiquement décrits dans la nomenclature internationale (ICSD3) comme Trouble des Rythmes Circadiens du Cycle Veille-Sommeil.

Il est aujourd'hui bien connu que la lumière permet de stimuler l'attention et les performances cognitives et motrices de l'homme, notamment en agissant sur les fonctions non-visuelles (veille, sommeil, humeur, horloge biologique). Le document US 2010/0217358 décrit par exemple la stimulation de l'éveil chez l'homme par de la lumière artificielle. Les documents JP2009283317 et WO201311589 décrivent des systèmes d'éclairage permettant d'améliorer les performances d'un utilisateur, ces systèmes modulant cycliquement la lumière du bleu au rouge à partir du spectre de lumière enrichi en bleu.

Or, il est aussi connu qu'un utilisateur étant exposé à un cycle de modulation de spectre de lumière commençant par une phase de quelques minutes de lumière enrichie en rouge augmente les performances cognitives par rapport à un cycle commençant par une phase de lumière enrichie en bleu, comme décrit par Mure et al. JBR 1999 et Chellapa et al. PNAS 2014.

Le document WO 2008/146220 décrit un dispositif d'éclairage pouvant générer un spectre variable pour créer un effet biologique. Le document EP 2 051 763 décrit un système et une méthode pour influencer un état photobiologique d'un sujet.

Si au cours d'une intervention chirurgicale, un utilisateur spécifique tel que le chirurgien entre dans un bloc opératoire présentant un système d'éclairage dont le cycle de modulation de spectre de lumière a déjà été initié et/ou que le système d'éclairage est en train d'émettre de la lumière dont le spectre est non-enrichi en rouge, la stimulation du chirurgien par la modulation de spectre de lumière sera moins efficace.

### Résumé de l'invention

Le but de l'invention est donc de proposer un équipement d'éclairage avec un dispositif d'éclairage à spectre accordable qui permet de renforcer les performances cognitives et psychomotrices du personnel médical en salle d'opération chirurgicale tout au long de la nuit et plus particulièrement à renforcer les performances du chirurgien dès lors qu'il pénètre dans une salle d'opération.

A cet effet, l'invention a pour objet un équipement d'éclairage notamment pour bloc opératoire comprenant un dispositif d'éclairage avec plusieurs sources de lumière pour fournir une lumière blanche à spectre lumineux accordable et un dispositif de contrôle/commande qui pilote les sources de lumière de façon à moduler le spectre lumineux selon un cycle de modulation répétable sans interruption comprenant une première période de temps où le spectre lumineux est enrichi en rouge, une seconde période de temps où le spectre lumineux est modulé graduellement du rouge vers le bleu, une troisième période de temps où le spectre lumineux est enrichi en bleu, une quatrième période de temps où le spectre lumineux est modulé graduellement du bleu vers le rouge caractérisé en ce que le dispositif de contrôle/commande est agencé pour détecter la réception d'un signal indicatif d'une présence humaine à proximité du dispositif d'éclairage et en ce qu'en réponse à la détection du signal, le dispositif de contrôle/commande réinitialise le cycle de modulation lorsque le cycle de modulation se trouve dans la première période de temps, ou module graduellement le spectre lumineux du bleu vers le rouge selon une période de temps transitoire lorsque le cycle de modulation se trouve dans la seconde, troisième ou quatrième période de temps avant de réinitialiser le cycle de modulation.

L'équipement d'éclairage selon l'invention peut encore présenter les particularités suivantes :
- la première période de temps est plus courte que la troisième période de temps ;
- la période de temps transitoire est plus courte que les seconde et quatrième périodes de temps ;
- les seconde et quatrième périodes de temps sont plus courtes que la troisième période de temps ;
- le dispositif de contrôle/commande est agencé pour commander les sources lumineuses de manière à maintenir un flux lumineux constant de lumière blanche pendant la variation du spectre lumineux de lumière blanche ;
- la proportion de rouge est plus importante que la proportion de bleu dans le spectre lumineux de lumière blanche pendant la première période de temps ;
- la proportion de bleu est plus importante que la proportion de rouge dans le spectre lumineux de lumière blanche pendant la troisième période de temps ;
- les proportions de rouge et de bleu dans le spectre lumineux de lumière blanche sont constantes respectivement durant la première période de temps et la troisième période de temps ;
- les proportions de bleu et de rouge dans le spectre lumineux de lumière blanche pendant la seconde période de temps passent graduellement des proportions de bleu et rouge présentes dans le spectre lumineux de lumière blanche pendant la première période de temps aux proportions de bleu et rouge présentes dans le spectre lumineux de lumière blanche pendant la troisième période de temps ;
- les proportions de bleu et de rouge dans le spectre lumineux de lumière blanche pendant la quatrième période de temps passent graduellement des proportions de bleu et de rouge présentes dans le spectre lumineux de lumière blanche pendant la troisième période de temps aux proportions de bleu et de rouge présentes dans le spectre lumineux de lumière blanche pendant la première période de temps ;
- il comprend au moins un capteur de présence connecté au dispositif de contrôle/commande pour recevoir le signal indicatif d'une présence humaine à proximité du dispositif d'éclairage ;
- le capteur de présence est un capteur sans contact pour recevoir un signal de type infrarouge, Bluetooth, ultra-son, radiofréquence ou vocale ;
- le capteur de présence est un capteur de vision avec reconnaissance faciale ;
- le capteur de présence est un capteur avec contact ;
- le capteur de présence reconnait une empreinte biométrique ;
- le capteur de présence est un lecteur de badge ou de puce ;
- il comprend deux capteurs de présence espacés l'un de l'autre ;
- le cycle de modulation répétable est programmable pour démarrer et / ou s'arrêter automatiquement à partir d'une heure prédéterminée de la journée ;
- le cycle de modulation répétable est activable manuellement par un utilisateur ;
- le cycle de modulation répétable est désactivable manuellement par un utilisateur ;
- les sources lumineuses sont des LEDs.

L'idée à la base de l'invention est donc de proposer un équipement d'éclairage antifatigue à modulation cyclique répétable de spectre lumineux afin de maximiser l'effet physiologique de la lumière et ainsi stimuler la vigilance, l'attention et les performances cognitives et psychomotrices du personnel médical tout au long de la nuit. L'effet de la lumière sur les fonctions non-visuelles passe principalement par l'activation des cellules ganglionnaires à mélanopsine de la rétine, qui présentent un pic de sensibilité dans le bleu (lambda max = 480 nm). Ainsi, une lumière enrichie en bleu est plus efficace qu'une lumière pauvre en bleu, en raison de la sensibilité des cellules ganglionnaires à mélanopsine.

Lors d'une exposition à la lumière, le photopigment des cellules à mélanopsine absorbe les photons, ce qui conduit à une réponse physiologique (activation des centres cérébraux impliqués dans la vigilance et la cognition). En même temps, ce processus désensibilise le photopigment et le rend inactif aux autres photons. Contrairement aux photopigments des cônes et des bâtonnets, qui nécessitent un processus de régénération long, la mélanopsine est capable de régénérer sa sensibilité par l'absorption d'un deuxième photon, dans la zone rouge du spectre. Cette réversibilité, appelée « bistabilité », permet ainsi à la mélanopsine de basculer entre les deux états, et d'être ainsi activée par la lumière « bleue » et réactivée par la lumière « rouge ».

C'est pourquoi l'alternance de lumière enrichie en rouge et bleue commençant par une première période de lumière enrichie en rouge maximise les effets stimulants de la lumière enrichie en bleue subséquente.

L'équipement d'éclairage de l'invention est donc conçu pour moduler cycliquement et sans interruption le spectre lumineux, d'une lumière à dominante rouge vers une lumière à dominante bleue de manière répétée. L'attention et les performances d'un chirurgien opérant de nuit ou dans des conditions de forte pression de sommeil, par exemple après de longues journées de travail, doivent tout particulièrement être stimulées. Ainsi, dès son entrée dans le bloc opératoire, le chirurgien doit être soumis la première période de temps d'exposition à la lumière enrichie en rouge afin d'amplifier les effets non visuels de la lumière enrichie en bleue à laquelle il sera exposé dans la troisième période de temps. C'est pourquoi dès que la présence du chirurgien est détectée à proximité du dispositif d'éclairage, si l'équipement d'éclairage est en train d'émettre de la lumière enrichie en rouge alors en réponse à cette détection le cycle de modulation est réinitialisé ; si l'équipement d'éclairage est en train d'émettre de la lumière enrichie en bleue ou un mélange de lumière enrichie en bleue/rouge, en réponse à cette détection le cycle de modulation est interrompu pour revenir graduellement à de la lumière enrichie en rouge pour redémarrer un cycle de modulation.

### Présentation sommaire des dessins

La présente invention sera mieux comprise et d'autres avantages apparaîtront à la lecture de la description qui suit et des dessins annexés dans lesquels :
- la figure 1 présente un ensemble d'éclairage selon un mode de réalisation de l'invention ;
- la figure 2 illustre deux cycles successifs de modulation du spectre lumineux ;
- la figure 3 présente trois graphiques représentant chacun un spectre lumineux au cours du cycle de modulation du spectre lumineux généré par l'ensemble d'éclairage selon l'invention ; sur chaque graphique de la figure 3, la longueur d'onde est en abscisse et la puissance radiométrique de la lumière normalisée à 100 est en ordonnée ;
- la figure 4 illustre le fonctionnement du dispositif de contrôle/commande.

### Description des modes de réalisation

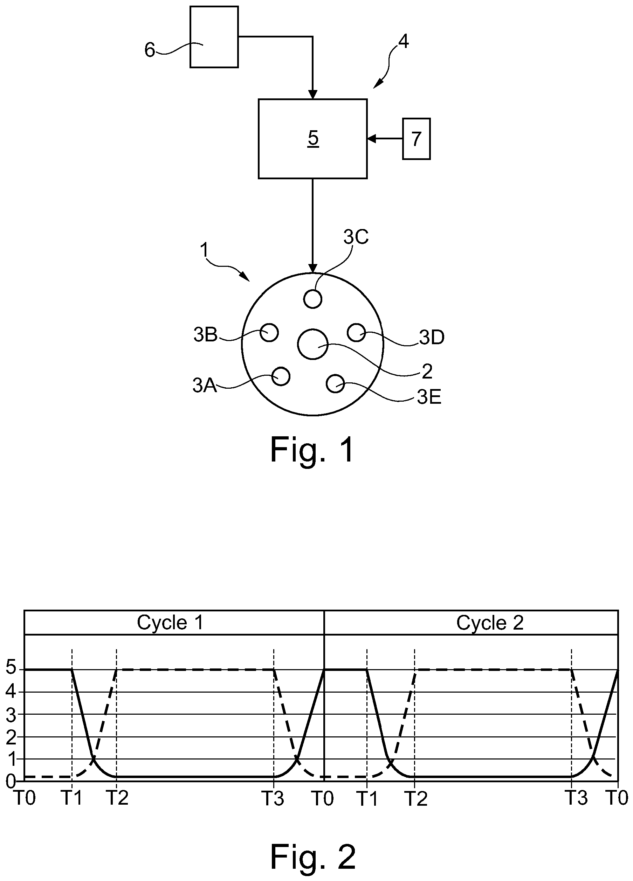

Sur la figure 1, l'ensemble d'éclairage à modulation de spectre lumineux selon l'invention comporte un dispositif d'éclairage 1, avec plusieurs sources de lumière, ici à diodes électroluminescentes ou LEDs 2, 3A à 3E. Le dispositif d'éclairage 1, peut être commandé au moyen d'un dispositif de contrôle/commande 4 électronique.

Plusieurs dispositifs d'éclairage tels que le dispositif d'éclairage 1 peuvent être implémentés dans l'ensemble d'éclairage encastré au plafond d'une pièce comme une salle opératoire, ou suspendu par exemple par l'intermédiaire d'un bras de suspension articulé.

Dans le dispositif d'éclairage 1, la LED 2 produit de la lumière blanche de température de couleur et d'IRC quelconque, la LED 3A produit de la lumière enrichie en bleue avec un pic de rayonnement compris entre 440 et 520 nm, la LED 3B produit de la lumière enrichie en rouge avec un pic de rayonnement compris entre 570 à 660 nm.

La LED 2 diffuse par exemple une lumière blanche à une température de couleur dans la zone de 4400K. Cette lumière blanche est mélangée selon l'invention avec la lumière produite par les LEDs 3A et 3B sous le contrôle du dispositif de contrôle/commande 4 qui pilote individuellement le courant d'alimentation dans ces LEDs.

Le dispositif de contrôle/commande 4 comporte notamment une unité de commande 5 du genre microprocesseur pilotée par l'utilisateur de l'ensemble d'éclairage via un bouton ou un clavier (non représenté) et une mémoire de programme 6.

Lors du choix du type d'ambiance lumineuse par l'utilisateur, l'unité de commande 5 déroule un programme enregistré dans la mémoire 6 pour piloter les LEDs 3A, 3B de sorte à générer plusieurs ambiances lumineuses correspondants à différents spectres lumineux de la lumière blanche produite par le dispositif d'éclairage 1.

En particulier, selon l'invention, l'unité de commande 5 fait suivre au spectre de lumière blanche un cycle de modulation répétable sans interruption. Le cycle de modulation comprend une période de temps où le spectre lumineux est enrichi en rouge et une période de temps où le spectre lumineux est enrichi en bleu, avec entre ces périodes une modulation graduelle du spectre lumineux du rouge vers le bleu ou du bleu vers le rouge.

Pour diminuer la fatigue de l'utilisateur, on utilise l'effet stimulateur de la lumière à dominante bleue tout au long de la nuit. Une lumière à dominante bleue, ponctuée par des périodes où la lumière est à dominante rouge, permet de régénérer les cellules ganglionnaires à mélanopsine. C'est pourquoi il est avantageux de pouvoir moduler cycliquement le spectre de lumière entre une dominante bleue et une dominante rouge. De plus, une exposition préliminaire à de la lumière enrichie en rouge, avant une exposition à de la lumière enrichie en bleue permet d'accroitre les effets de la lumière enrichie en bleue sur les fonctions non-visuelles de l'utilisateur. Ainsi selon l'invention, dès l'allumage de l'ensemble d'éclairage, le cycle de modulation du spectre de lumière commence par une période d'exposition à de la lumière enrichie en rouge.

La figure 2 présente deux cycles successifs de modulation des composantes bleue et rouge du spectre lumineux, le trait plein représente le ratio rouge sur bleu, et le trait en pointillé représente le ratio bleu sur rouge. A l'initialisation T0 du cycle, le spectre lumineux est enrichi en rouge et restera enrichi en rouge durant une première période de temps T0-T1 qui peut durer par exemple entre 2 minutes et 10 minutes.

La première période de temps T0-T1 est suivie d'une seconde période de temps T1-T2 pendant laquelle le spectre lumineux passe graduellement du rouge au bleu. Afin de ne pas perturber le confort visuel des utilisateurs, la deuxième période de temps T1-T2 peut durer 5 minutes par exemple.

En fin de seconde période de temps T1-T2 le spectre lumineux est enrichi en bleu. Il le restera durant une troisième période T2-T3 qui peut s'étendre par exemple entre 10 minutes et 20 minutes.

Une quatrième période de temps T3-T0 est la dernière étape d'un cycle de modulation durant laquelle le spectre lumineux passe graduellement du bleu au rouge, en 5 minutes par exemple. Une fois qu'un cycle se termine, un deuxième peut s'enchainer automatiquement et ainsi de suite.

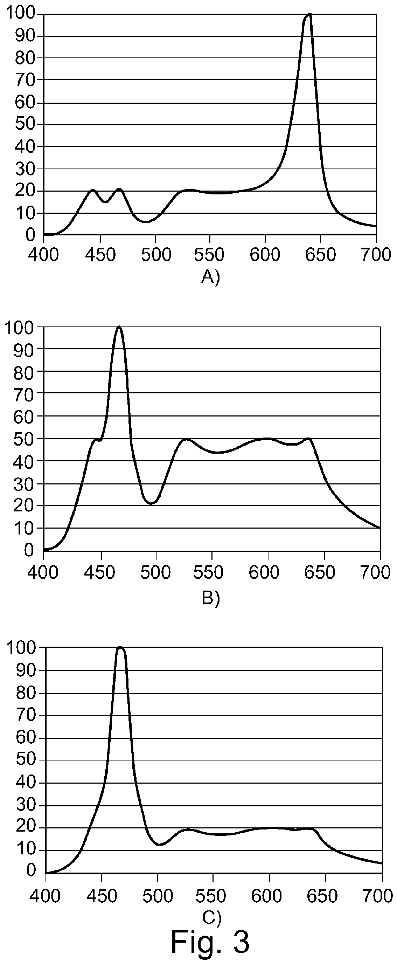

Lorsque le spectre lumineux passe du rouge au bleu, ou du bleu au rouge, pour que le changement ne soit pas perceptible par l'utilisateur et n'incommode pas son confort, l'unité de commande 5 peut générer des transitions graduelles du spectre, du bleu vers le rouge et du rouge vers le bleu. Ainsi l'unité de commande 5 génère une multitude de spectres intermédiaires entre le spectre enrichi en bleu et le spectre enrichi en rouge et inversement, comme cela sera illustré dans la figure 3. Chaque spectre intermédiaire est proche des deux spectres qui lui sont adjacents et est généré sur une durée minimale d'observation pour l'œil de l'utilisateur.

Selon l'invention, un cycle tel que décrit précédemment peut durer entre 10 minutes et plusieurs heures, la durée optimale étant comprise entre 22 et 40 minutes. De préférence, la première période de temps T0-T1 est plus courte que ladite troisième période de temps T2-T3. Les seconde T1-T2 et quatrième T3-T0 périodes de temps sont plus courtes que la troisième période de temps.

Le graphique A) de la figure 3, montre un spectre rouge généré lors de la première période de temps du cycle de modulation. Le graphique B) de la figure 3, montre un spectre intermédiaire entre le rouge et le bleu généré pendant la deuxième période au cours de laquelle la modulation est graduelle. Le graphique C) de la figure 3 montre un spectre bleu généré pendant la troisième période de temps du cycle de modulation.

On doit comprendre que pendant la seconde période de temps T1-T2 du cycle de modulation, l'unité de commande 5 décrémente graduellement la proportion de rouge tout en augmentant graduellement la proportion de bleu. Durant la quatrième période de temps T3-T0 du cycle de modulation, l'unité de commande 5 augmente graduellement la proportion de rouge et décrémente graduellement la proportion de bleu.

Au cours d'une opération chirurgicale, un chirurgien doit tout particulièrement être attentif et performant. C'est pourquoi, la stimulation visuelle offerte par l'ensemble d'éclairage selon l'invention doit être optimale pour le chirurgien dès son arrivée dans la salle opératoire. Ainsi, si le chirurgien pénètre dans le bloc opératoire alors que l'ensemble d'éclairage émet de la lumière enrichie en bleue ou un mélange de lumière enrichie en bleue/rouge, les effets d'amplification des réponses non visuelles à la lumière bleue seront moindres pour le chirurgien. Il est alors important de pouvoir identifier le chirurgien et détecter sa présence afin qu'il reçoive une stimulation visuelle optimale dès son entrée dans le bloc opératoire.

Pour cela, selon l'invention l'équipement d'éclairage comprend un capteur de présence 7 connecté au dispositif de contrôle/commande 4. Ce capteur de présence 7 est apte à recevoir un signal indicatif d'une présence humaine telle que la présence d'une certaine personne, ici le chirurgien. Le capteur de présence 7 peut être un capteur sans contact qui reçoit un signal par infrarouge, par Bluetooth, par ultra-son ou par radiofréquence, ou un capteur avec contact tel qu'un bouton mécanique sur lequel le chirurgien appuie, ou encore un capteur qui reconnaîtrait une empreinte biométrique telle qu'une empreinte digitale.

Le capteur peut être un capteur de vision pour de la reconnaissance faciale, mais aussi un capteur pour de la reconnaissance vocale.

Le capteur peut être un lecteur de carte ou de puce.

Dans le cas par exemple d'utilisation de la localisation d'une puce RFID, un capteur de présence peut être placé au sol aux seuils des portes ou du sas du bloc opératoire. Afin de déterminer le sens de passage du chirurgien, deux capteurs peuvent être positionnés, un dans le bloc et un dans le couloir. Dans ce cas, la partie identification du chirurgien pourra être placée dans sa chaussure, dans sa semelle par exemple. Sans restreindre la portée de l'invention, d'autres capteurs peuvent être ajoutés afin d'améliorer la détermination du sens de passage du chirurgien.

Dans un autre mode de réalisation, une table d'opération peut aussi être équipée d'un capteur RFID pour détecter la présence du chirurgien dans son rayon d'action.

Selon l'invention, dès la détection d'un signal d'entrée du chirurgien dans le bloc opératoire, si l'équipement d'éclairage est en train d'émettre de la lumière enrichie en rouge alors l'unité de commande 5 du dispositif de contrôle/commande 4 réinitialise le cycle de modulation; si l'équipement d'éclairage est en train d'émettre de la lumière enrichie en bleue ou un mélange de lumière enrichie en bleue/rouge, le cycle de modulation est interrompu pour revenir graduellement selon une période de temps transitoire à de la lumière enrichie en rouge pour redémarrer un cycle de modulation normal. On comprendra que durant cette période de temps transitoire, l'unité de commande 5 augmente graduellement la proportion de rouge et décrémente graduellement la proportion de bleu. Néanmoins, pour que le chirurgien ne soit pas exposé à un spectre initial non enrichi en rouge, cette période de temps transitoire doit être courte.

Dans le cas ou le chirurgien est amené à sortir du bloc opératoire, le signal indicatif de sa présence à proximité du dispositif d'éclairage ne sera plus détecté mais le cycle de modulation continuera normalement. Si le chirurgien entre à nouveau dans le bloc opératoire, le dispositif de contrôle/commande 4 détectera à nouveau un signal de sa présence et la procédure décrite ci-dessus sera reproduite.

Dans un autre mode de réalisation, plusieurs chirurgiens pourraient être identifiables et chacun enverrait un signal indicatif de sa présence à proximité du dispositif d'éclairage.

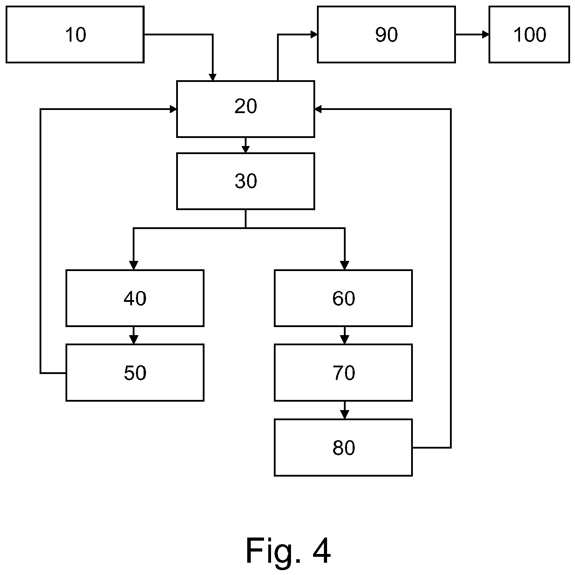

La figure 4 illustre le fonctionnement de l'unité de commande 5. Elle est démarrée en 10 par actionnement du clavier par exemple. L'unité de commande 5 est programmée pour se caler sur le spectre à dominante rouge quand l'ensemble d'éclairage est actionné et à l'étape 20 elle génère le premier cycle de modulation que le signal indicatif de la présence du chirurgien soit reçu ou non par le capteur de présence 7 connecté à l'unité de commande 5. Elle répète ce cycle de modulation en boucle, par exemple pendant toute la durée de l'intervention, toute la nuit, et arrête ce processus si par exemple elle reçoit une commande d'arrêt de fonctionnement ou de changement de mode de fonctionnement.

Si à l'étape 30 le dispositif de contrôle/commande 4 détecte la réception d'un signal indicatif de la présence du chirurgien à proximité du dispositif d'éclairage 1, alors selon l'invention :
- si le spectre lumineux à l'étape 40 est enrichi en rouge, l'unité de commande 5 commande à l'étape 50 la réinitialisation du cycle de modulation sans modification du spectre lumineux pour se retrouver à l'étape 20.
- si le spectre lumineux à l'étape 60 est enrichi en bleu ou est un mélange de bleu/rouge, alors l'unité de commande 5 commande à l'étape 70 une transition graduelle du spectre lumineux émis vers le spectre lumineux enrichi en rouge, puis commande la réinitialisation à l'étape 80 du cycle de modulation du spectre lumineux pour se retrouver à l'étape 20.

L'étape 90 correspond au moment où le capteur de présence ne reçoit plus de signal, le cycle de modulation continue jusqu'à l'arrêt de l'équipement d'éclairage ou jusqu'à la désactivation possible du programme.

A l'étape 70, correspondant à la période de temps transitoire énoncée précédemment, il est avantageux d'augmenter la vitesse de transition par rapport à la vitesse normale de modulation graduelle, c'est-à-dire lors d'un cycle de modulation non perturbé. Cette augmentation de vitesse, pendant cette période de temps transitoire, ne doit pas perturber le travail et le confort du personnel médical déjà présent dans le bloc opératoire tout en permettant de diminuer un temps d'exposition au spectre non rouge initial du chirurgien. Cette période de temps transitoire est généralement plus courte que les seconde T1-T2 et quatrième T3-T0 périodes de temps.

Il est à noter qu'un mode de fonctionnement, manuel ou programmé, peut être désactivé manuellement au profit d'un autre mode de fonctionnement, permettant par exemple de choisir une couleur particulière de la lumière émise.

Lorsqu'un mode de fonctionnement est plus particulièrement destiné aux travailleurs de nuit, il peut être programmé pour se désactiver le jour, de 7 heure à 19 heure par exemple et s'activer automatiquement à partir d'une certaine heure, 19 heure par exemple. Néanmoins, si un utilisateur désire activer ce mode de fonctionnement en cours de journée, il peut le faire.

Selon l'invention, les différents spectres d'éclairage peuvent être enregistrés dans la mémoire de programme 6 sous une forme exploitable par l'unité de commande 5 pour piloter le courant dans les LEDs 2, 3A, 3B.

En plus des lumières bleue et rouge produites par les LEDs 3A et 3B, on peut aussi mélanger à la lumière blanche des lumières jaune, verte, et cyan par exemple produites par d'autres LEDs 3C, 3D, 3E représentées sur la figure 1. Ces LEDs 3C à 3E sont aussi pilotées par l'unité de commande 5 et servent par exemple à améliorer l'indice de rendu des couleurs.

Par exemple, sur le graphique A) de la figure 3 la couleur d'enrichissement est le rouge. On voit que le rapport de puissance radiométrique entre le bleu et le rouge est de 20%. Pareillement, le rapport de puissance radiométrique entre le vert et le rouge est de 20%.

Sur le graphique C) de la figure 3 on voit en ordonnées que la puissance radiométrique du vert représente 20% de la puissance radiométrique du bleu à 100% qui est ici la couleur d'enrichissement. On voit également sur le graphique C) que la puissance radiométrique du rouge représente 20% de la puissance radiométrique du bleu.

Sur le graphique B) on voit la transition pour passer d'un spectre à dominante rouge à un spectre à dominante bleu tout en conservant une puissance radiométrique d'au moins 20% entre la longueur d'onde de la composante de couleur d'enrichissement et chaque longueur d'onde des autres composantes de couleur dans le spectre de la lumière blanche.

Pour conserver un bon rendu des couleurs, il est entendu que les lumières produites par les LEDs 3C, 3D, 3E sont modulées graduellement pour suivre l'incrémentation de la composante rouge ou bleu dans le cycle.

A noter que l'unité de commande 5 est agencée pour maintenir une puissance d'éclairage constante indépendamment du cycle de modulation de spectre de la lumière émise par l'ensemble d'éclairage par exemple à l'aide d'un capteur de flux lumineux (non représenté) disposé dans le dispositif d'éclairage 1 et qui renvoie une mesure de flux pour asservir le pilotage du courant dans les LEDs. Ainsi, le dispositif de contrôle/commande (4) commande les sources de lumières (2, 3A-3E) de manière à maintenir un flux lumineux constant de lumière blanche pendant la modulation du spectre lumineux de lumière blanche.

Il va de soi que la présente invention ne saurait être limitée à la description qui précède d'un de ses modes de réalisation, susceptibles de subir quelques modifications sans pour autant sortir du cadre de l'invention.

## Revendications

1. Equipement d'éclairage antifatigue notamment pour bloc opératoire comprenant un dispositif d'éclairage (1) avec plusieurs sources de lumière (2, 3A-3E) pour fournir une lumière blanche à spectre lumineux accordable et un dispositif de contrôle/commande (4) qui pilote lesdites sources de lumière de façon à moduler ledit spectre lumineux selon un cycle de modulation répétable sans interruption comprenant une première période de temps (T0-T1) où ledit spectre lumineux est enrichi en rouge, une seconde période de temps (T1-T2) où ledit spectre lumineux est modulé graduellement du rouge vers le bleu, une troisième période de temps (T2-T3) où ledit spectre lumineux est enrichi en bleu, une quatrième période de temps (T3-T0) où ledit spectre lumineux est modulé graduellement du bleu vers le rouge **caractérisé en ce que** au moins un capteur de présence (7) connecté audit dispositif de contrôle/commande (4) reçoit un signal indicatif d'une détection d'une présence à proximité dudit dispositif d'éclairage (1) et transmet ladite détection dudit signal audit dispositif de contrôle/commande (4) et **en ce qu'**en réponse à ladite détection dudit signal, ledit dispositif de contrôle/commande (4) réinitialise ledit cycle de modulation lorsque ledit cycle de modulation se trouve dans ladite première période de temps (T0-T1), ou module graduellement ledit spectre lumineux du bleu vers le rouge selon une période de temps transitoire lorsque ledit cycle de modulation se trouve dans ladite seconde (T1-T2), troisième (T2-T3) ou quatrième (T3-T0) période de temps avant de réinitialiser ledit cycle de modulation.

2. Equipement d'éclairage selon la revendication 1, **caractérisé en ce que** ladite première période de temps (T0-T1) est plus courte que ladite troisième période de temps (T2-T3).

3. Equipement d'éclairage selon la revendication 1, **caractérisé en ce que** ladite période de temps transitoire est plus courte que lesdites seconde (T1-T2) et quatrième (T3-T0) périodes de temps.

4. Equipement d'éclairage selon la revendication 1, **caractérisé en ce que** lesdites seconde (T1-T2) et quatrième (T3-T0) périodes de temps sont plus courtes que ladite troisième période de temps (T2-T3).

5. Equipement d'éclairage selon la revendication 1, **caractérisé en ce que** ledit dispositif de contrôle/commande (4) est agencé pour commander lesdites sources de lumières (2, 3A-3E) de manière à maintenir un flux lumineux constant de lumière blanche pendant ladite modulation dudit spectre lumineux de lumière blanche.

6. Equipement d'éclairage selon la revendication 1, **caractérisé en ce que** la proportion de rouge est plus importante que la proportion de bleu dans ledit spectre lumineux de lumière blanche pendant ladite première période de temps (T0-T1).

7. Equipement d'éclairage selon la revendication 1, **caractérisé en ce que** la proportion de bleu est plus importante que la proportion de rouge dans ledit spectre lumineux de lumière blanche pendant ladite troisième période de temps (T2-T3).

8. Equipement d'éclairage selon la revendication 1, **caractérisé en ce que** les proportions de rouge et de bleu dans ledit spectre lumineux de lumière blanche sont constantes respectivement durant ladite première période de temps (T0-T1) et ladite troisième période de temps (T2-T3).

9. Equipement d'éclairage selon la revendication 1, **caractérisé en ce que** les proportions de bleu et de rouge dans ledit spectre lumineux de lumière blanche pendant ladite seconde période de temps (T1-T2) passent graduellement des proportions de bleu et rouge présentes dans ledit spectre lumineux de lumière blanche pendant ladite première période de temps (T0-T1) aux proportions de bleu et rouge présentes dans ledit spectre lumineux de lumière blanche pendant ladite troisième période de temps (T2-T3).

10. Equipement d'éclairage selon la revendication 1, **caractérisé en ce que** les proportions de bleu et de rouge dans ledit spectre lumineux de lumière blanche pendant ladite quatrième période de temps (T3-T0) passent graduellement des proportions de bleu et de rouge présentes dans ledit spectre lumineux de lumière blanche pendant ladite troisième période de temps (T2-T3) aux proportions de bleu et de rouge présentes dans ledit spectre lumineux de lumière blanche pendant ladite première période de temps (T0-T1).

11. Equipement d'éclairage selon la revendication 1, **caractérisé en ce que** ledit capteur de présence (7) est un capteur sans contact pour recevoir un signal de type infrarouge, Bluetooth, ultra-son, radiofréquence ou vocale.

12. Equipement d'éclairage selon la revendication 1, **caractérisé en ce que** ledit capteur de présence (7) est un capteur de vision avec reconnaissance faciale.

13. Equipement d'éclairage selon la revendication 1, **caractérisé en ce que** ledit capteur de présence (7) est un capteur avec contact.

14. Equipement d'éclairage selon la revendication 13, **caractérisé en ce que** ledit capteur de présence (7) reconnait une empreinte biométrique.

15. Equipement d'éclairage selon la revendication 1, **caractérisé en ce que** ledit capteur de présence (7) est un lecteur de badge ou de puce.

16. Equipement d'éclairage selon la revendication 1, **caractérisé en ce qu'**il comprend deux capteurs de présence (7) espacés l'un de l'autre.

17. Equipement d'éclairage selon la revendication 1, **caractérisé en ce que** le cycle de modulation répétable est programmable pour démarrer et / ou s'arrêter automatiquement à partir d'une heure prédéterminée de la journée.

18. Equipement d'éclairage selon la revendication 1, **caractérisé en ce que** le cycle de modulation répétable est activable manuellement par un utilisateur.

19. Equipement d'éclairage selon la revendication 1, **caractérisé en ce que** le cycle de modulation répétable est désactivable manuellement par un utilisateur.

20. Equipement d'éclairage selon l'une quelconque des revendications précédentes, dans lequel lesdites sources lumineuses (2, 3A-3E) sont des LEDs.

## Patentansprüche

1. Beleuchtungsausrüstung gegen Ermüdung, insbesondere für Operationsblock, umfassend eine Beleuchtungsvorrichtung (1) mit mehreren Lichtquellen (2, 3A-3E) zum Verschaffen eines weißen Lichts mit anpassbaren Lichtspektren und eine Regel-/Steuervorrichtung (4), die die Lichtquellen derart steuert, dass das Lichtspektrum gemäß einem wiederholbaren Modulationszyklus ohne Unterbrechung moduliert wird, umfassend einen ersten Zeitraum (T0-T1), während dem das Lichtspektrum in Rot angereichert wird, einen zweiten Zeitraum (T1-T2), während dem das Lichtspektrum schrittweise vom Roten zum Blauen moduliert wird, einen dritten Zeitraum (T2-T3), während dem das Lichtspektrum in Blau angereichert wird, einen vierten Zeitraum (T3-T0), während dem das Lichtspektrum schrittweise vom Blauen zum Roten moduliert wird,
**dadurch gekennzeichnet,**
**dass** wenigstens ein mit der Regel-/Steuervorrichtung (4) verbundener Anwesenheits-Sensor (7) ein Signal empfängt, das für die Erfassung einer Anwesenheit in der Nähe der Beleuchtungsvorrichtung (1) repräsentativ ist und diese Erfassung des Signals der Regel-/Steuervorrichtung (4) zuleitet, und
**dass** in Reaktion auf die Erfassung des Signals die Regel-/Steuervorrichtung (4) den Modulationszyklus reinitialisiert, wenn der Modulationszyklus sich in dem ersten Zeitraum (T0-T1) befindet, oder, wenn der Modulationszyklus sich in dem zweiten (T1-T2), dem dritten (T2-T3) oder dem vierten (T3-T0) Zeitraum befindet, gemäß eines Übergangszeitraums schrittweise das Lichtspektrum vom Blauen zum Roten moduliert wird, bevor der Modulationszyklus reinitialisiert wird.

2. Beleuchtungsvorrichtung nach Anspruch 1, **dadurch gekennzeichnet, dass** der erste Zeitraum (T0-T1) kürzer ist als der dritte Zeitraum (T2-T3).

3. Beleuchtungsvorrichtung nach Anspruch 1, **dadurch gekennzeichnet, dass** der Übergangszeitraum kürzer ist als die zweiten (T1-T2) und vierten (T3-T0) Zeiträume.

4. Beleuchtungsvorrichtung nach Anspruch 1, **dadurch gekennzeichnet, dass** die zweiten (T1-T2) und vierten (T3-T0) Zeiträume kürzer sind als der dritte Zeitraum (T2-T3).

5. Beleuchtungsvorrichtung nach Anspruch 1, **dadurch gekennzeichnet, dass** die Regel-/Steuervorrichtung (4) ausgebildet ist, um die Lichtquellen (2, 3A-3E) derart zu steuern, dass ein konstanter Lichtfluss von weißem Licht während der Modulation des Lichtspektrums des weißen Lichts beibehalten wird.

6. Beleuchtungsvorrichtung nach Anspruch 1, **dadurch gekennzeichnet, dass** während des ersten Zeitraums (T0-T1) in dem Lichtspektrum des weißen Lichts der Anteil von Rot größer ist als der Anteil von Blau.

7. Beleuchtungsvorrichtung nach Anspruch 1, **dadurch gekennzeichnet, dass** während des dritten Zeitraums (T2-T3) in dem Lichtspektrum des weißen Lichts der Anteil von Blau größer ist als der Anteil von Rot.

8. Beleuchtungsvorrichtung nach Anspruch 1, **dadurch gekennzeichnet, dass** jeweils während des ersten Zeitraums (T0-T1) und des dritten Zeitraums (T2-T3) in dem Lichtspektrum des weißen Lichts die Anteile von Rot und Blau konstant sind.

9. Beleuchtungsvorrichtung nach Anspruch 1, **dadurch gekennzeichnet, dass** während des zweiten Zeitraums (T1-T2) in dem Lichtspektrum des weißen Lichts die Anteile von Blau und Rot schrittweise von den Anteilen von Blau und Rot, die in dem Lichtspektrum des weißen Lichts während des ersten Zeitraums (T0-T1) vorhanden sind, zu den Anteilen von Blau und Rot, die in dem Lichtspektrum des weißen Lichts während des dritten Zeitraums (T2-T3) vorhanden sind, übergehen.

10. Beleuchtungsvorrichtung nach Anspruch 1, **dadurch gekennzeichnet, dass** während des vierten Zeitraums (T3-T0) in dem Lichtspektrum des weißen Lichts die Anteile von Blau und Rot schrittweise von den Anteilen von Blau und Rot, die in den Lichtspektren des weißen Lichts während des dritten Zeitraums (T2-T3) vorhanden sind, zu den Anteilen von Blau und Rot, die in dem Lichtspektrum des weißen Lichts während des ersten Zeitraums (T0-T1) vorhanden sind, übergehen.

11. Beleuchtungsvorrichtung nach Anspruch 1, **dadurch gekennzeichnet, dass** der Anwesenheits-Sensor (7) ein kontaktloser Sensor ist zum Empfangen eines Signals des Typs Infrarot, Bluetooth, Ultraschall, Radiofrequenz oder akustisch.

12. Beleuchtungsvorrichtung nach Anspruch 1, **dadurch gekennzeichnet, dass** der Anwesenheits-Sensor (7) ein Bildsensor mit Gesichtserkennung ist.

13. Beleuchtungsvorrichtung nach Anspruch 1, **dadurch gekennzeichnet, dass** der Anwesenheits-Sensor (7) ein Sensor mit Kontakt ist.

14. Beleuchtungsvorrichtung nach Anspruch 13, **dadurch gekennzeichnet, dass** der Anwesenheits-Sensor (7) einen biometrischen Abdruck erkennt.

15. Beleuchtungsvorrichtung nach Anspruch 1, **dadurch gekennzeichnet, dass** der Anwesenheits-Sensor (7) ein Lesegerät für Abzeichen oder Mikrochips ist.

16. Beleuchtungsvorrichtung nach Anspruch 1, **dadurch gekennzeichnet, dass** sie zwei voneinander beabstandete Anwesenheits-Sensoren (7) umfasst.

17. Beleuchtungsvorrichtung nach Anspruch 1, **dadurch gekennzeichnet, dass** der wiederholbare Modulationszyklus programmierbar ist zum automatischen Starten und/oder Anhalten ausgehend von einer vorbestimmten Zeit des Tages.

18. Beleuchtungsvorrichtung nach Anspruch 1, **dadurch gekennzeichnet, dass** der wiederholbare Modulationszyklus manuell durch einen Benutzer aktivierbar ist.

19. Beleuchtungsvorrichtung nach Anspruch 1, **dadurch gekennzeichnet, dass** der wiederholbare Modulationszyklus manuell durch einen Benutzer deaktivierbar ist.

20. Beleuchtungsvorrichtung nach einem der vorhergehenden Ansprüche, **dadurch gekennzeichnet, dass** die Lichtquellen (2, 3A-3E) LEDs sind.

## Claims

1. Anti-fatigue lighting equipment, in particular for an operating theater, which lighting equipment comprises a lighting device (1) with a plurality of light sources (2, 3A-3E) for delivering white light having a tunable light spectrum, and a monitoring and control device (4) that controls said light sources in such a manner as to modulate said light spectrum in a repeatable modulation cycle without interruption, which cycle comprises a first period of time (T0-T1) during which said light spectrum is red-enriched, a second period of time (T1-T2) during which said light spectrum is gradually modulated from red to blue, a third period of time (T2-T3) during which said light spectrum is blue-enriched, and a fourth period of time (T3-T0) during which said light spectrum is modulated gradually from blue to red, said lighting equipment being **characterized in that** at least one presence sensor (7) connected to said monitoring and control unit (4) receives a signal indicative of a presence in the vicinity of said lighting device (1) and transmits said detection of said signal to said monitoring and control device (4), and **in that**, in response to said signal being detected, said monitoring and control device (4) reinitializes said modulation cycle when said modulation cycle is in said first period of time (T0-T1), or gradually modulates said light spectrum from blue to red during a transient period of time when said modulation cycle is in said second (T1-T2), third (T2-T3) or fourth (T3-T0) period of time before reinitializing said modulation cycle.

2. Lighting equipment according to claim 1, **characterized in that** said first period of time (T0-T1) is shorter than said third period of time (T2-T3).

3. Lighting equipment according to claim 1, **characterized in that** said transient period of time is shorter than said second (T1-T2) and third (T3-T0) periods of time.

4. Lighting equipment according to claim 1, **characterized in that** said second (T1-T2) and fourth (T2-T3) periods of time are shorter than said third period of time (T2-T3).

5. Lighting equipment according to claim 1, **characterized in that** said monitoring and control device (4) is arranged to control said light sources (2, 3A-3E) in such a manner as to maintain a constant flux of white light during said modulation of said light spectrum of white light.

6. Lighting equipment according to claim 1, **characterized in that** the proportion of red is greater than the proportion of blue in said light spectrum of white light during said first period of time (T0-T1).

7. Lighting equipment according to claim 1, **characterized in that** the proportion of blue is greater than the proportion of red in said light spectrum of white light during said third period of time (T2-T3).

8. Lighting equipment according to claim 1, **characterized in that** the proportions of red and blue in said light spectrum of white light are constant respectively during said first period of time (T0-T1) and during said third period of time (T2-T3).

9. Lighting equipment according to claim 1, **characterized in that** the proportions of blue and red in said light spectrum of white light during said second period of time (T1-T2) go gradually from the proportions of blue and red that are present in said light spectrum of white light during said first period of time (T0-T1) to the proportions of blue and red that are present in said light spectrum of white light during said third period of time (T2-T3).

10. Lighting equipment according to claim 1, **characterized in that** the proportions of blue and red in said light spectrum of white light during said fourth period of time (T3-T0) go gradually from the proportions of blue and red that are present in said light spectrum of white light during said third period of time (T2-T3) to the proportions of blue and red that are present in said light spectrum of white light during said first period of time (T0-T1).

11. Lighting equipment according to claim 1, **characterized in that** said presence sensor (7) is a contactless sensor for receiving a signal of the infrared, Bluetooth, ultra-sound, radiofrequency or voice type.

12. Lighting equipment according to claim 1, **characterized in that** said presence sensor (7) is a vision sensor with facial recognition.

13. Lighting equipment according to claim 1, **characterized in that** said presence sensor (7) is a contact sensor.

14. Lighting equipment according to claim 3, **characterized in that** said presence sensor (7) recognizes a biometric signature.

15. Lighting equipment according to claim 1, **characterized in that** said presence sensor (7) is a badge or chip reader.

16. Lighting equipment according to claim 1, **characterized in that** it includes two presence sensors (7) spaced apart from each other.

17. Lighting equipment according to claim 1, **characterized in that** the repeatable modulation cycle is programmable to start and/or to stop automatically as from a predetermined time of the day.

18. Lighting equipment according to claim 1, **characterized in that** the repeatable modulation cycle is activatable manually by a user.

19. Lighting equipment according to claim 1, **characterized in that** the repeatable modulation cycle is deactivatable manually by a user.

20. Lighting equipment according to any preceding claim, wherein said light sources (2, 3A-3E) are LEDs.
